# FASCICULE DE BREVET EUROPEEN

(11) **EP 0 808 139 B1**
(45) Date de publication et mention de la délivrance du brevet: **30.06.1999**
(21) Numéro de dépôt: 96901664.1
(22) Date de dépôt: 06.02.1996
(51) Int. Cl.: A61F 2/06

(54) **PROTHESE INTRA-AORTIQUE ET INSTRUMENTATION CHIRURGICALE DESTINEE A L'INTRODUCTION, LA MISE EN PLACE ET LA FIXATION DE CETTE PROTHESE DANS L'AORTE**
INTRAAORTALE PROTHESE UND CHIRURGISCHE VORRICHTUNG ZUM EINBRINGEN, POSITIONIEREN UND BEFESTIGEN DIESER PROTHESE IN DER AORTA
INTRA-AORTIC PROSTHESIS AND SURGICAL INSTRUMENTS FOR INSERTING, POSITIONING AND FIXING SAID PROSTHESIS IN THE AORTA

(30) Priorité: 10.02.1995 BE 9500106
(43) Date de publication de la demande: 26.11.1997
(73) Titulaire: De Fays, Robert, 6700 Arlon (Fouches) (BE); Deldime, Paul, 6700 Arlon (BE)
(72) Inventeur: De Fays, Robert, 6700 Arlon (Fouches) (BE); Deldime, Paul, 6700 Arlon (BE)
(74) Mandataire: Callewaert, Jean
(86) Numéro de dépôt international: BE9600008
(87) Numéro de publication internationale: WO9624306

(56) Documents cités:
- EP-A- 0 461 791
- EP-A- 0 508 473
- EP-A- 0 539 237
- WO-A-94/04097
- US-A- 3 657 744
- US-A- 4 562 596
- US-A- 4 994 071

## Description

La présente invention est relative à une prothèse intra-aortique pour le traitement, par voie endovasculaire, d'anévrisme de l'aorte abdominale, notamment au niveau du carrefour aortique, tel que défini dans le préambule de la revendication 1.

Une telle prothèse a été décrite dans le document EP-A-539237.

Actuellement, par suite de l'évolution technologique, il est possible de traiter des sténoses artérielles athéromateuses par des méthodes radiologiques interventionnelles dites d'angioplastie percutanée, avec des résultats de plus en plus favorables.

La pathologie anévrismale de l'aorte reste très particulière puisque cette maladie se définit par une dilatation pathologique d'origine multifactorielle évoluant inéluctablement vers la rupture.

Le traitement en reste alors chirurgical pur avec les résultats aléatoires bien connus.

Toutefois, avant la rupture, le traitement chirurgical électif des anévrismes peut être considéré comme efficace sur le plan de la mortalité mais est grevé d'une morbidité et d'un coût financier importants d'autant plus que ce traitement s'adresse généralement à des patients âgés dont, dans la plupart des cas, les autres artères sont pratiquement toujours malades à des degrés divers.

Le traitement chirurgical appliqué jusqu'à présent par voie endovasculaire dans le cas d'un anévrisme de l'aorte abdominale consiste à insérer, via une incision dans l'artère fémorale, une prothèse au moyen d'une instrumentation appropriée vers l'anévrisme à traiter. Une telle intervention chirurgicale est généralement très complexe, surtout lorsque l'anévrisme n'est pas limitée à l'aorte abdominale mais s'étend également dans les deux artères iliaques. En effet, dans un tel cas il y a lieu de réaliser un lien suffisamment étanche, à l'endroit du carrefour aortique, entre une prothèse introduite par une des artères iliaques et celle introduite par l'artère iliaque opposée.

Il s'est avéré, dans la pratique, que les moyens techniques existant actuellement ne permettent pas toujours de réaliser cette opération avec la précision requise.

Un des buts essentiels de la présente invention est de présenter une prothèse intra-aortique permettant de remédier aux inconvénients des techniques existantes pour le traitement d'anévrismes de l'aorte abdominale, surtout au niveau du carrefour aortique.

A cet effet, la prothèse intra-aortique, suivant l'invention, est caractérisée par la partie caractérisante de la revendication 1

Suivant une forme de réalisation particulièrement avantageuse de l'invention, un stent en forme d'un treillis expansible, réalisé en un métal à mémoire, est agencé à l'intérieur du tube, de préférence à proximité de l'extrémité libre de ce dernier.

L'invention est également relative à une instrumentation chirurgicale destinée à permettre la mise en place, à l'endroit désiré, d'une prothèse intraaortique, notamment de la prothèse telle que décrite ci-dessus, comprenant des moyens permettant la mise en place de cette prothèse.

Cette instrumentation est caractérisée par le fait qu'elle comprend les instruments suivants : (a) un manchon d'introduction préformé en double courbure sensiblement en forme de "S" destiné à être engagé, à partir d'un accès pratiqué dans l'artère sous-clavière gauche vers l'aorte en épousant la configuration du confluent aortico-sous-clavier gauche, (b) au moins un guide réalisé en une matière radio-opaque et de préférence recouverte d'une substance à propriétés lubrifiantes en présence d'humidité, (c) une gaine semi-rigide destinée à être engagée dans le manchon d'introduction susdit et l'aorte sur une distance s'étendant à partir de l'artère sous-clavière gauche au moins jusqu'à proximité de l'ostium des artères rénales, (d) au moins un cathéter-poussoir et (e) un piston, dont la section extérieure correspond sensiblement à la section intérieure de la gaine, pouvant s'appuyer sur la prothèse engagée dans la gaine et muni d'une tige de commande semi-rigide pouvant actionner le piston. Une instrumentation selon le préambule de la revendication 8 est connue de EP-A-461791.

D'autres détails et particularités de l'invention ressortiront de la description donnée ci-après, à titre d'exemple non-limitatif, d'une forme de réalisation particulière d'une prothèse intra-aortique et d'une instrumentation chirurgicale, suivant l'invention, avec référence aux dessins annexés.
La figure 1 est une vue schématique en perspective et de face de la prothèse suivant cette forme de réalisation particulière.
La figure 2 est une vue schématique en perspective latérale de cette prothèse.
La figure 3 est une représentation schématique montrant cette prothèse mise en place dans l'aorte abdominale et les artères iliaques primitives.
La figure 4 est une vue schématique illustrant l'emplacement d'un manchon d'introduction dans l'artère sous-clavière gauche.
La figure 5 est une vue schématique d'ensemble en perspective et avec brisure partielle montrant la mise en place d'une prothèse intra-aortique en faisant usage de l'instrumentation chirurgicale suivant cette forme de réalisation particulière de l'invention.
La figure 6 est, à plus grande échelle, une représentation schématique partielle en élévation d'une partie essentielle de la figure 5.
La figure 7 est une vue schématique partielle en perspective d'un détail de la partie essentielle suivant la figure 6.
La figure 8 est, à une échelle différente, une représentation schématique d'une section transversale suivant la ligne VIII-VIII de la figure 6.

Dans les différentes figures les mêmes chiffres de référence sont relatifs aux mêmes éléments.

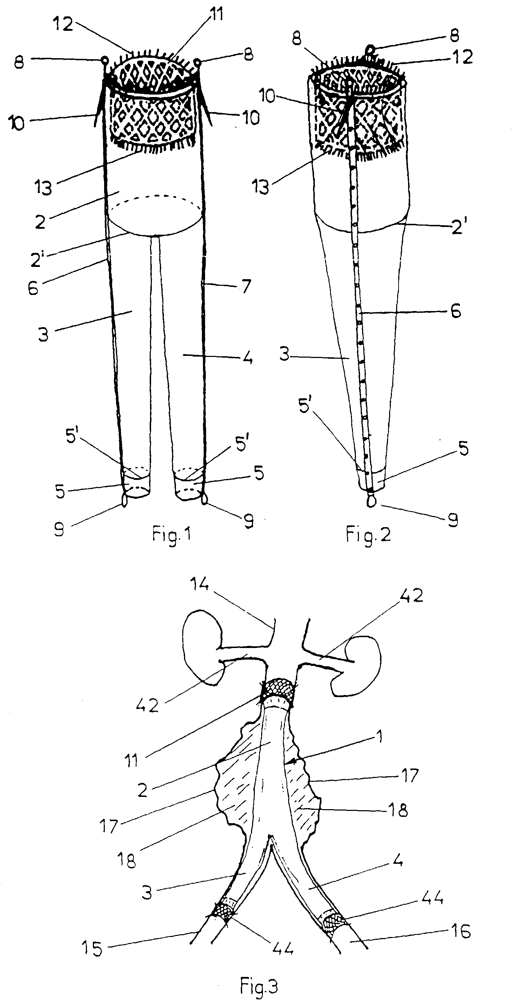

La forme de réalisation particulière de la prothèse intra-aortique 1, suivant l'invention, telle que représentée aux figures 1 et 2 pour le traitement, par voie endovasculaire, d'anévrisme de l'aorte abdominale au niveau du carrefour aortique est réalisée en une matière flexible et médicalement acceptable, notamment du polytétrafluoroéthylène, connue sous le nom commercial "Teflon" et obtenue par extrusion et expansion. Cette prothèse 1 est essentiellement formée d'un tube 2 bifurquant à une de ses extrémités en deux branches tubulaires 3 et 4 de section plus réduite que celle du tube 2.

Comme montré clairement aux figures 1 et 2, cette prothèse 1 présente ainsi la forme d'une culotte, de sorte qu'elle peut être identifiée comme "culotte aortoiliaque".

L'épaisseur de la paroi de cette prothèse 1 est comprise entre 0,10 et 0,3 mm, de préférence entre 0,15 et 0,20 mm.

Le tube 2 présente une longueur de l'ordre de 55 mm et a une texture connue sous le nom de "strecht", dont l'orientation des fibres est telle à permettre une extension radiale du tube 2 lors de l'application d'une pression à l'intérieur de ce dernier. Ainsi, un tube présentant au repos un diamètre proximal de 25 mm peut être amené à 30 mm. A titre de variante, si le diamètre est au repos de 20 mm, celui-ci peut être amené à 25 mm lors de l'application d'une pression intérieure.

Les branches tubulaires 3 et 4 sont de préférence cousues à l'extrémité 2' du tube 2 à partir de laquelle elles s'étendent et sont sensiblement inextensibles sur au moins la plus grande partie de leur longueur.

Suivant l'application envisagée, elles présentent une longueur de 9 cm pour le positionnement dans une artère iliaque primitive ou de 14 cm pour un positionnement dans une artère iliaque externe.

De plus, la zone de bord annulaire 5, d'une hauteur de l'ordre d'au moins 1 cm, est, comme le tube 2, d'une texture permettant l'extension du diamètre de cette zone de 8 à 12 mm. Cette zone 5 est formée d'un tube séparé qui est cousu sur l'extrémité libre 5' de ces branches 3 et 4.

Par ailleurs, sur la face extérieure de la prothèse 1 ainsi confectionnée sont fixées deux bandes de raidissement continues 6 et 7 qui s'étendent parallèlement à l'axe du tube 2, en des endroits diamètralement opposés, et ceci sur toute la longueur de ce tube 2 et de chacune des deux branches 3 et 4. Dans cette forme de réalisation particulière, ces bandes 6 et 7 sont formées par des lames ajourées en acier inoxydable, d'une largeur de l'ordre de 1 mm et d'une épaisseur de l'ordre de 0,1 mm. Ces lames se terminent à leurs deux extrémités par des oeillets 8 et 9 dans lesquels peuvent être passés en boucle simple des fils de suspension et des fils de traction, comme il sera décrit ci-après.

En plus de ces oeillets 8 et 9, sur chacune des lames 8 et 9, à proximité des oeillets 8, est soudé un crampon 10 expansible latéralement d'une longueur de par exemple 3 à 5 mm ou 5 à 8 mm. Dans certains cas, il peut être utile de prévoir deux de tels crampons 10 sur chacune de ces lames 6 et 7.

A l'intérieur du tube 2, à proximité de son extrémité libre ou proximale est prévu un treillis 11 de forme cylindrique réalisé en un métal à mémoire et présentant une structure telle à être auto-expansible. Ce treillis a une longueur maximale de 5 cm et un diamètre déployé de l'ordre de 20 à 30 mm. De plus, il présente à ses deux bords, en état déployé, des crampons obliques 12 et 13 orientés vers l'extérieur. Les crampons 12 situés le long du bord libre du tube 1 sont orientés vers le haut et s'étendent à l'extérieur de la prothèse, tandis que les crampons 13 au bord opposé du treillis sont destinés à s'ancrer dans la prothèse 1 même. Un tel treillis est généralement appelé "stent".

Le treillis 11 est cousu au tube 2, uniquement à l'endroit de la paroi intérieure de ce dernier opposé aux lames 6 et 7, de manière à permettre ainsi de replier la prothèse 1 facilement autour de son axe longitudinal et d'obtenir ainsi un volume de la prothèse en direction radiale aussi réduite que possible.

La figure 3 est une représentation schématique de la façon dont la prothèse 1 est positionnée dans l'aorte 14 et les deux artères iliaques primitives 15 et 16 à un endroit où se présente un anévrisme 17.

La prothèse étant très légèrement poreuse, cette dernière sera pénétrée par du sang qui coagulera dans l'espace 18 entre les parties dilatées de l'aorte 14 et la prothèse 1.

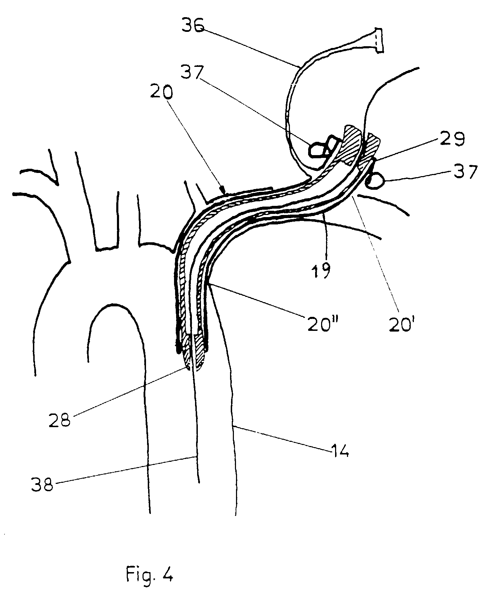

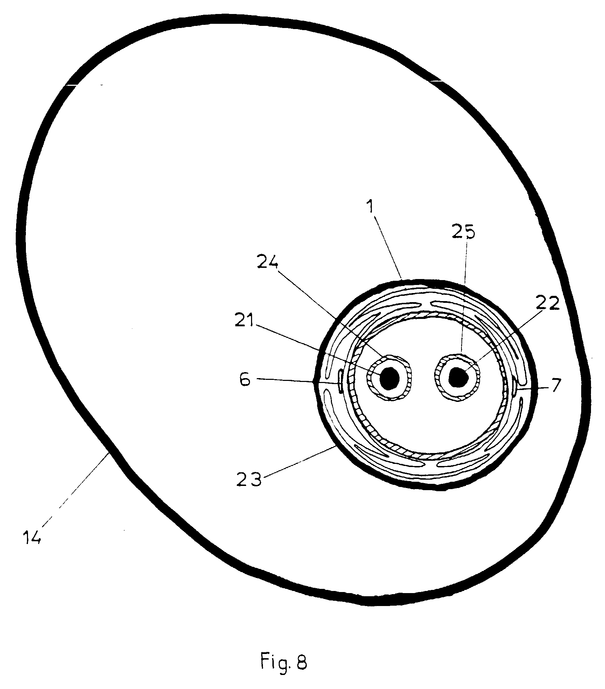

Les figures 4 à 8 illustrent la technique chirurgicale mise en oeuvre pour l'introduction, le positionnement exact et la fixation sur place de la prothèse 1 en faisant usage d'une instrumentation chirurgicale particulière qui fait également l'objet de la présente invention.

Cette instrumentation comprend les instruments suivants : (a) un manchon d'introduction 19 destiné à être introduit dans l'artère sous-clavière gauche 20, (b) deux guides 21 et 22 en une matière radio-opaque recouverts d'une substance, connue en soi, à propriétés lubrifiantes en présence d'humidité, (c) une gaine semi-rigide 23 destinée à être engagée dans le manchon d'introduction 19 et l'aorte 14, (d) deux cathéters-poussoirs 24 et 25 et (e) un piston 26, dont la section extérieure correspond sensiblement à la section intérieure de la gaine 23, muni d'une tige de commande 27 semi-rigide destinée à actionner le piston 26.

Le manchon d'introduction 19, encore appelé "introducteur sous-clavier gauche", est réalisé en polytétrafluoroéthylène semi-rigide, connu sous le nom commercial "Teflon". Il présente un diamètre externe de maximum 7,3 mm et une longueur de l'ordre de 30 cm.

Comme montré clairement à la figure 4, ce manchon 19 est préformé en double courbure sensiblement en forme de "S" de manière à permettre de l'engager à partir d'un accès 20' pratiqué dans l'artère sous-clavière gauche 20 vers l'aorte 14 en épousant la configuration du confluent aortico-sous-clavier gauche 20''.

Par ailleurs, il présente des oeillets 37 permettant de le fixer à la peau dès sa mise en place, cette dernière étant controlée, d'une manière connue en soi, par une injection de produits opaques par un conduit latéral 36 aboutissant dans son extrémité distale.

Concrètement, par artériotomie sous-clavière controlée chirurgicalement, ce manchon 19 est introduit en lui appliquant un mouvement de rotation jusqu'à être engagé dans l'aorte descendante. A cet égard, il contient avantageusement un mandrin flexible 28, en une matière plastique médicalement acceptable, coulissant sur un guide radio-opaque 38, dont l'extrémité distale est arrondie et relativement molle pour faciliter son introduction sans risque de blessures, tout en assurant l'étanchéité nécessaire.

Ensuite, après le retrait du mandrin, son extrémité proximale est fermée par une valve étanche 29 pouvant présenter trois trous de passage dont la fonction sera décrite ci-après.

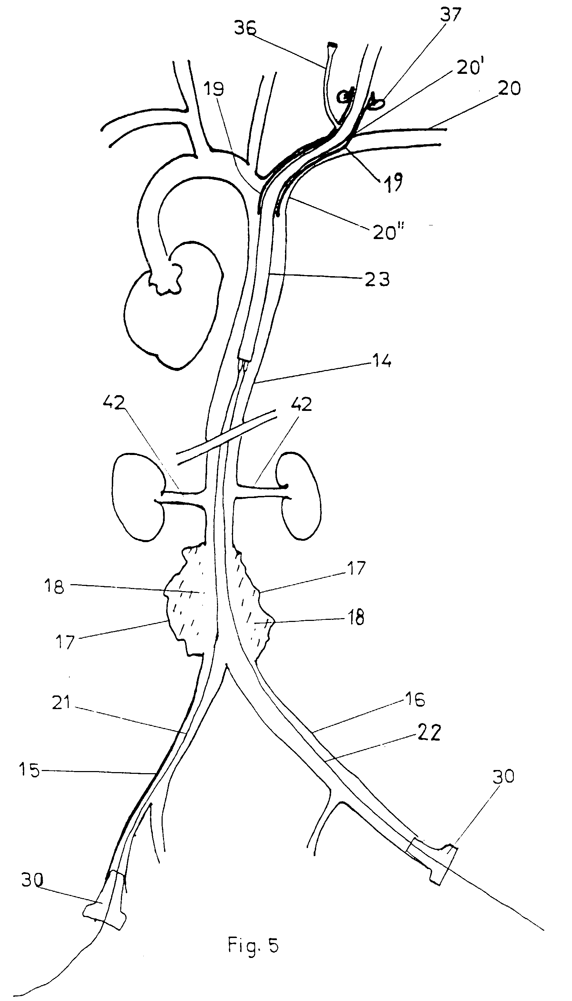

Un dispositif d'introduction 30, appelé "introducteur fémoral" à valve French 9, connu en soi, est prévu pour ressortir les guides 21 et 22 par des insertions pratiquées dans chacune des artères iliaques 15 et 16, comme montré à la figure 5.

Ces guides présentent de préférence un diamètre maximum de l'ordre de 0,035 pouce et une longueur de l'ordre de 260 cm.

La gaine 23 est de préférence réalisée en polytétrafluoroéthylène semi-rigide, d'un diamètre extérieur ne dépassant avantageusement pas 6,8 mm et d'une longueur par exemple d'au moins 160 cm.

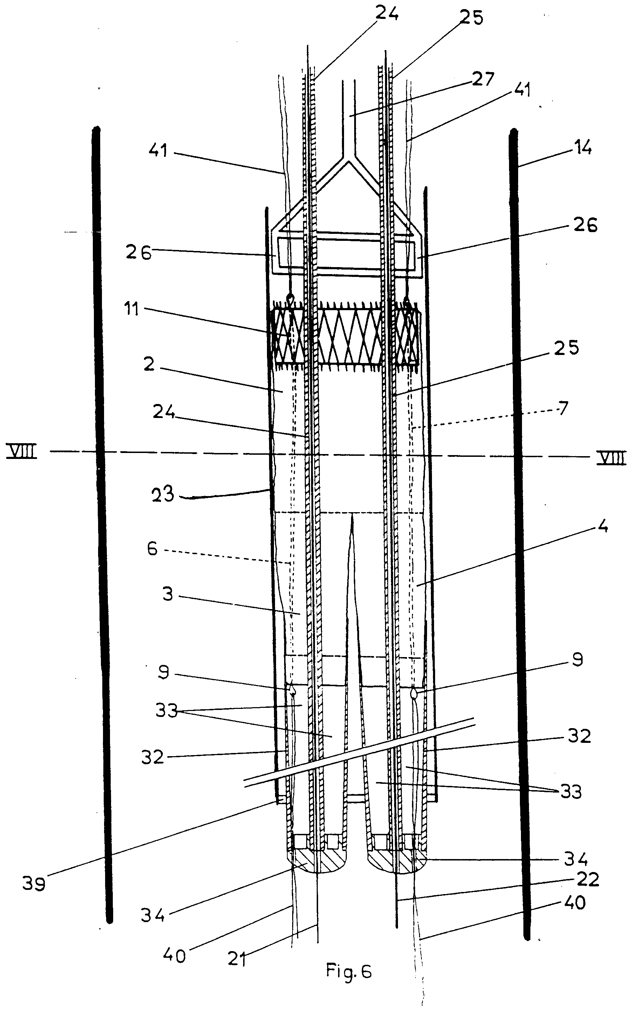

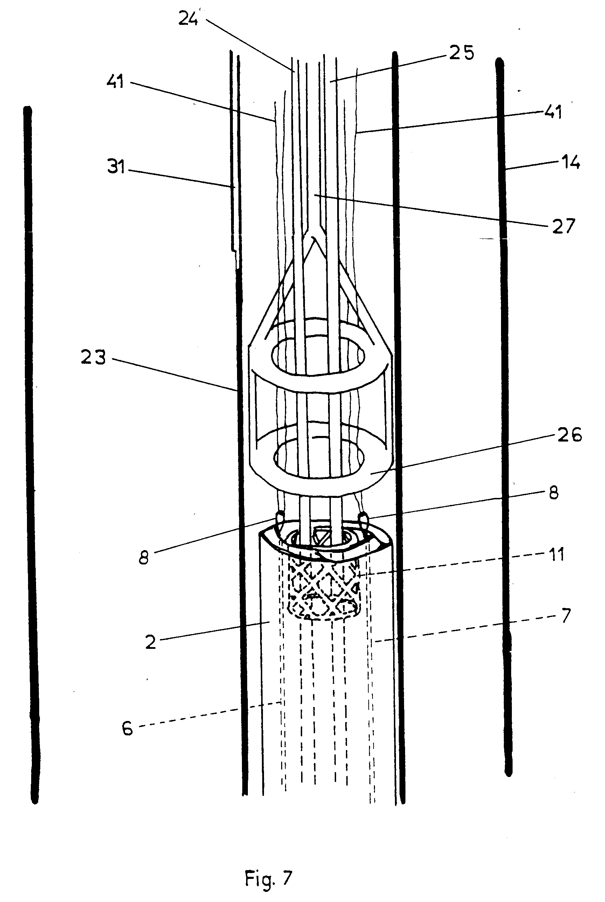

Cette gaine 23 contient dans son épaisseur un conduit 31 relié à un cathéter (figure 7), tandis qu'un repère radio-opaque 39 (figure 6) est prévu à son extrémité distale.

Comme montré à la figure 6, chacun des cathéters-poussoirs 24 et 25 est pourvu d'une capsule 32 destinée à recevoir l'extrémité distale des deux branches 3 et 4 de la prothèse 1.

Cette capsule 32 est formée par une garniture tubulaire s'étendant à partir de l'extrémité libre de ces cathéters-poussoirs sur une certaine hauteur et à une certaine distance, latéralement et extérieurement autour de ces derniers, de manière à permettre de maintenir lesdites extrémités des branches 3 et 4 de la prothèse 1 fermement dans l'espace 33 ménagé entre ces cathéters-poussoirs et les capsules 32.

Par ailleurs, un bouchon annulaire 34, dont l'extrémité distale est arrondie, peut être engagé dans la capsule 32 autour de l'extrémité distale de chacun des cathéters-poussoirs 24 et 25 pour solidariser la capsule de son cathéter-poussoir correspondant et empêcher que du sang puisse pénétrer dans l'espace entre les cathéters-poussoirs et les capsules et ainsi entrer en contact avec la prothèse. Ce bouchon 34 ne peut dépasser latéralement de la capsule. De plus, des fils de traction 40 enfilés dans les oeillets 9 sont tirés à travers le bouchon 34.

Ci-après est donnée une description de la façon suivant laquelle la prothèse 1 est introduite dans l'aorte 14 et positionnée à l'endroit d'un névrisme 17.

D'abord un accès 20' est pratiqué dans l'artère sous-clavière et une ponction fémorale est réalisée en utilisant les introducteurs French 9 à valve 30 placés par la méthode connue de Seldinger.

La méthode Seldinger est une méthode très connue, généralement appliquée en chirurgie vasculaire, pour l'introduction percutanée dans une veine ou une artère d'une électrode, d'un ballon ou d'un cathéter en vue d'angiographie. A cet égard, on fait usage d'une instrumentation comprenant une gaine avec valve anti-retour, un dilatateur et un fil mini-guide.

Ensuite, on introduit par artériotomie sous-clavière gauche l'introducteur 19 également selon la même méthode de Seldinger et sous contrôle radiologique.

Une fois ces introducteurs 19 et 30 mis en place les deux guides 21 et 22 sont engagés dans l'introducteur 19, après avoir sorti le mandrin 28 avec le guide 38 de ce dernier, dans l'aorte 14 et glissés vers chacun des artères iliaques 15 et 16 pour ressortir par l'introducteur fémoral correspondant 30.

Après ces travaux préparatoires, la gaine 23, contenant les deux cathéters-poussoirs 24 et 25 avec leurs capsules de protection respectives 32, la prothèse, repliée 1 sur le stent 11 et autour des cathéters-poussoirs 24 et 25, et le piston 26 s'appuyant sur l'extrémité proximale de la prothèse, est engagée dans l'introducteur 19.

Avant l'introduction dans la gaine 23, la prothèse 1 est enfilée sur les cathéters-poussoirs 24 et 25, de manière à ce qu'un des cathéters 24 s'étende dans une des branches 3 de cette dernière et le second cathéter 25 dans l'autre branche 4. De plus, l'extrémité distale de chacune de ces branches 3 et 4 est maintenue par la capsule correspondante 32 contre l'extrémité distale de ce cathéter et les fils de traction 40 et de suspension 41 sont enfilés en boucle dans leurs oeillets respectifs 8 et 9.

Ensuite, la gaine 23 et l'ensemble de son contenu, tel que décrit ci-dessus, sont glissés sur les deux guides radiologiques 21 et 22 en passant ces derniers à l'intérieur des cathéters-poussoirs et ceci jusqu'à 3 à 4 cm de la bifurcation iliaque.

Les branches 3 et 4 de la prothèse 1 sont ainsi descendues sur chacun de ces guides 21 et 22 en poussant conjointement le piston 26 et les deux cathéters-poussoirs 24 et 25 et ceci jusqu'à ce que l'extrémité de chaque capsule 32 ressorte au niveau fémoral. Une fois que l'extrémité proximale ait par exemple passé l'ostium des artères rénales 42, la gaine 23 est progressivement retirée par l'accès 20' dans l'artère sous-clavière gauche 20 jusqu'à ce que l'extrémité distale du stent 11 se superpose sur le repère radiologique 39 marquant l'extrémité inférieure de la gaine 23.

La prothèse 1 est retenue dans cette position, d'une part, en maintenant les fils de suspension 41 passés dans les oeillets proximaux 8 fixés à l'extrémité proximale des lames 6 et 7 et, d'autre part, en mettant les fils de traction 40 sous tension à partir de l'accès dans les artères iliaques 15 et 16. Ces fils de traction 40 étant solidarisés de la capsule 32 de chacune des cathéters-poussoirs, les extrémités distales de la prothèse 1 peuvent être séparées de cette capsule en agissant sur ces fils de traction au moment où la prothèse est en place, comme montré à la figure 3.

Avantageusement, comme montré à la figure 6, les extrémités libres des branches 3 et 4 s'étendent à une distance de l'ordre de 30 cm du fond des capsules 32. Ceci a comme résultat que les capsules se présentent aux introducteurs fémoraux 44 au moment où la prothèse 1 atteint sa position exacte dans l'aorte 14 et les artères iliaques 15 et 16. Ceci permet donc l'accès facile à ces capsules pour leur retrait.

Les cathéters-poussoirs 24 et 25 avec leurs capsules respectives 32 sont alors ressortis complètement par voie fémorale tout en maintenant sous traction les fils 40 passés dans les oeillets 9 des lames 7 et 8 de la prothèse 1.

Avantageusement, pour faciliter l'enlèvement de la capsule, celle-ci est conçue pour être fendue longitudinalement lors de son retrait.

Dans une étape suivante, la gaine 23 est retirée par l'abord sous-clavier permettant de positionner le stent 11 contenu dans le tube 2 de la prothèse 1 en-dessous des artères rénales 42 et de contrôler sa position par une injection de produit opaque par le conduit latéral 31 de la gaine 23 en veillant à ce qu'au moins la partie proximale du tube 2 avec le stent 11 reste contenue dans la gaine 23.

Ensuite, sur un des guides radiologiques 24 ou 25 et par l'abord sous-clavier 20 un nouveau cathéter avec un ballon aortique, non représenté aux figures mais connu en lui-même, est glissé dans la gaine 23 jusqu'à l'intérieur du stent 11 et y est gonflé à une pression de l'ordre de O,5 atmosphère.

La gaine 23 est alors retirée suffisamment par l'abord sous-clavier 20 pour libérer ainsi le stent auto-expansible 11.

A ce moment, le ballon aortique est gonflé à une pression de 2 à 3 atmosphères permettant ainsi d'ancrer, dans la paroi aortique, les crampons 12 du stent 11 en dépliant en même temps complètement la prothèse 1, pendant que les crampons 13 s'ancrent dans celle-ci. De cette façon, la prothèse prend la forme telle que représentée à la figure 3.

Le ballon aortique est ensuite dégonflé et, au moyen d'une opacification radiologique, la qualité de l'étirement de la prothèse 1 est contrôlée, celle-ci étant constamment maintenue sous tension au moyen des fils de traction 40 et de suspension 41.

Par chacun des abords fémoraux et sur chacune des guides 21 et 22 un stent 44, analogue au stent 11 mais de dimensions plus réduites, est positionné dans la zone distale radialement extensible 5 de chacune des branches 3 et 4 de la prothèse 1, en exerçant en même temps une traction sur les fils 40 afin d'empêcher les branches 3 et 4 de remonter.

Par un nouveau contrôle radiologique l'étirement et le positionnement de la prothèse par traction sur les fils fémoraux 40 sont vérifiés.

Les stents 44 sont libérés et, par une opacification générale, on contrôle le fonctionnement de la prothèse 1 avant d'enlever les fils fémoraux de traction 40 et sous-claviers de suspension 41.

Ensuite, la gaine 23 contenant le piston 26, ainsi que l'introducteur 19 sont retirés complètement, tandis que les guides 21 et 22 sont rentrés dans l'artère sous-clavière, de manière à permettre de procédér à la fermeture de l'artériotomie sous-clavière gauche et de son abord chirurgical.

Enfin, les guides 21 et 22 sont retirés, après une dernière vérification radiologique, par les introducteurs fémoraux 30, qui sont ensuite également enlevés.

Comme il résulte de ce qui précède, un des grands avantages de la prothèse et de l'instrumentation suivant l'invention est qu'il est possible d'introduire aussi bien la prothèse que pratiquement toute l'instrumentation dans le sens du courant sanguin. Grâce à la forme de cette prothèse, elle est adaptée à la configuration anatomique naturelle, indépendamment de l'ampleur de l'anévrisme ou de son extension sur les artères iliaques en ceci en évitant les difficultés d'un abord à contre-courant.

Par ailleurs, l'instrumentation, suivant l'invention, permet une insertion parfaite sous contrôle radiologique sans rencontrer des difficultés de torsion, de plicature et/ou de lâchage.

A titre illustratif, ci-après est donné un exemple concret des spécifications de l'instrumentation, suivant l'invention, décrite ci-dessus:
a) introducteur sous clavier 19 :
   - diamètre intérieur : 6,7 mm
   - diamètre extérieur : 7,3 mm
   - longueur : 15 cm
b) gaine 23 :
   - diamètre intérieur : 6,3 mm
   - diamètre extérieur : 6,7 mm
   - longueur : 80 cm
c) guides 21 et 22 :
   - longueur : 260 cm
   - diamètre : 0,035 pouce
d) cathéters-poussoirs 24 et 25 (X 2) :
   - longueur : 120 cm
      (X1) diamètre : 1,3 mm
      (X2) diamètre : ± 3 mm
e) stent auto-extensible 11 :
   - diamètre maximum : 30 mm
   - longueur : 50 mm.

Il est important de noter que les mesures données ci-dessus sont des valeurs maximum et généralement une préférence est donnée à une valeur inférieure pour ce qui concerne la largeur et la section de certains des instruments, notamment en fonction de nature des matières instruments, notamment en fonction de nature des matières utilisées, les techniques de fabrication, le névrisme à traiter et l'état de l'aorte et des artères du patient.

L'invention n'est, toutefois, pas limitée à la forme de réalisation particulière de la prothèse et l'instrumentation, telles que décrites ci-dessus et représentées aux dessins annexés, mais dans le cadre de l'invention, différentes variantes peuvent être envisagées.

Ainsi, l'instrumentation, suivant l'invention, peut parfaitement convenir pour l'introduction et la mise en place d'une prothèse en forme de tuyau, par exemple, si l'anévrisme se limite à l'aorte abdominale.

Par ailleurs, la prothèse 1, la gaine 23 et/ou l'introducteur 19 peuvent être réalisés en une autre matière médicalement acceptable que le polytétrafluoroéthylène.

## Revendications

1. Prothèse intra-aortique (1), réalisée en une matière flexible et médicalement acceptable, pour le traitement, par voie endovasculaire, d'anévrisme (17) de l'aorte abdominale (14), notamment au niveau du carrefour aortique, comprenant un tube (2) bifurquant à une de ses extrémités en deux branches tubulaires (3) et (4) de section plus réduite que celle du tube (2), et un treillis (11) de forme cylindrique, réalisé en un métal à mémoire et présentant une structure telle à être auto-expansible dans le tube (2), caractérisée en ce que deux bandes de raidissement (6) et (7) continues s'étendent parallèlement à l'axe du tube (2), sur les côtés latéraux extérieurs et diamétralement opposés de ce dernier, sur sensiblement toute sa longueur et se prolongeant également sur sensiblement toute la longueur des deux branches (3) et (4), ces bandes de raidissement (6) et (7) présentant à chacune de leurs extrémites des oeillets (8) et (9) dans lesquels peuvent être fixés respectivement des fils de suspension (41) et des fils de traction (40).

2. Prothèse (1) suivant la revendication 1, caractérisée en ce que les bandes de raidissement précitées (6) et (7) sont formées par des lames métalliques.

3. Prothèse (1) suivant l'une ou l'autre des revendications 1 et 2, caractérisée en ce que, à l'extrémité proximale de chacune des bandes de raidissement (6) et (7), à l'extérieur de ces dernières et à proximité des oeillets (8), est fixé un crampon (10) expansible latéralement et orienté vers l'extrémité opposée ou distale de ces bandes de raidissement (6) et (7).

4. Prothèse suivant l'une quelconque des revendications 1 à 3, caractérisée en ce que les bandes de raidissement (6) et (7) sont ajourées sur sensiblement toute leur longueur.

5. Prothèse suivant l'une quelconque des revendications 1 à 4, caractérisée en ce que les bandes de raidissement sont cousues sur le tube (2) et les deux branches tubulaires (3) et (4).

6. Prothèse suivant l'une quelconque des revendications 1 à 5, caractérisée en ce que le treillis précité (11) est uniquement fixé au tube (2) à l'endroit de la paroi intérieure de ce dernier opposé aux bandes de raidissement (6) et (7).

7. Prothèse suivant l'une quelconque des revendications 1 à 6, caractérisée en ce qu'au moins les branches (3) et (4) sont sensiblement inextensibles alors que leur extrémité libre présente une zone annulaire (5) extensible radialement.

8. Instrumentation chirurgicale comprenant des moyens permettant la mise en place d'une prothèse intra-aortique selon l'une des revendications 1 à 7, caractérisée en ce qu'elle comprend au moins un des instruments suivants :
(a) un manchon d'introduction (19), de préférence, préformé en double courbure sensiblement en forme de "S" destiné à être engagé à partir d'un accès pratiqué dans l'artère sous-clavière gauche (20) vers l'aorte (14) en épousant la configuration du confluent aortico-sous-clavier gauche, (b) au moins un guide (21) et (22) réalisé en une matière radio-opaque et de préférence recouverte d'une substance à propriétés lubrifiantes en présence d'humidité, (c) une gaine (23) semi-rigide destinée à être engagée dans le manchon d'introduction susdit et l'aorte (14) jusqu'à proximité de l'ostium des artères rénales, (d) au moins un cathéter-poussoir (24, 25) et (e) un piston (26), dont la section extérieure correspond sensiblement à la section intérieure de la gaine (23), pouvant s'appuyer sur la prothèse (1) engagée dans la gaine (23) et muni d'une tige de commande semi-rigide (27) pouvant actionner le piston (26).

9. Instrumentation suivant la revendication 8, caractérisée en ce que le manchon d'introduction (19) est réalisé en polytétrafluoroéthylène.

10. Instrumentation suivant l'une ou l'autre des revendications 8 et 9, caractérisée en ce qu'elle comprend un mandrin (28), en une matière flexible, destiné à être monté dans le manchon d'introduction précité (19), ce mandrin (28) présentant une extrémité distale arrondie et étant percé de manière à pouvoir coulisser sur un guide radio-opaque (38) tout en assurant l'étanchéité nécessaire.

11. Instrumentation suivant l'une quelconque des revendications 8 à 10, caractérisée en ce qu'elle comprend une valve (29) destinée à être engagée dans l'extrémité proximale du manchon (19) et présentant des passages pour la tige précitée (27) du piston (26) et le cathéter-poussoir (24, 25).

12. Instrumentation suivant l'une quelconque des revendications 8 à 11, caractérisée en ce qu'elle comprend, pour chacun des artères iliaques (15, 16), un tuyau d'introduction (30) à valve French 9 pour ressortir les guides précités introduits via l'artère sous-clavière gauche (20) dans l'aorte (14).

13. Instrumentation suivant l'une quelconque des revendications 8 à 12, caractérisée en ce que la gaine (23) est réalisée en polytétrafluoroéthylène semi-rigide.

14. Instrumentation suivant l'une quelconque des revendications 8 à 13, caractérisé en ce que l'extrémité distale de la gaine (23) est pourvue d'un repaire radiologique (39).

15. Instrumentation suivant l'une quelconque des revendications 8 à 14, caractérisée en ce qu'un conduit (31) relié à un cathéter est incorporé dans la paroi de la gaine (23) permettant la création d'une opacification radiologique au-dessus du piston (26) et la prothèse (1) qu'elle contient.

16. Instrumentation suivant l'une quelconque des revendications 8 à 15, caractérisée en ce que le cathéter-poussoir (24-25) est agencé de manière à pouvoir servir de conduite pour le guide précité (21, 22).

17. Instrumentation suivant l'une quelconque des revendications 8 à 16, caractérisée en ce qu'elle comprend deux cathéters-poussoirs (24, 25) destinés à être introduits dans chacune des branches (3) et (4) de la prothèse précitée (1).

18. Instrumentation suivant l'une quelconque des revendications 8 à 17, caractérisée en ce que l'extrémité distale du cathéter-poussoir (24,25) est pourvue d'une capsule (32) destinée à recevoir l'extrémité distale de la prothèse (1).

19. Instrumentation suivant la revendication 18, caractérisée en ce que la capsule (32) est formée par une garniture tubulaire s'étendant à une certaine distance, latéralement et extérieurement autour de l'extrémité distale du cathéter-poussoir (24, 25), de manière à permettre de maintenir l'extrémité distale de la prothèse dans l'espace (33) ménagé entre le cathéter-poussoir (24, 25) et la capsule (32).

20. Instrumentation suivant la revendication 19, caractérisée en ce qu'elle comprend un bouchon annulaire (34) à extrémité distale arrondie destiné à maintenir, d'une part, la capsule (32) sensiblement coaxialement autour de l'extrémité distale du cathéter-poussoir (24, 25) et, d'autre part, des fils de traction 40 fixés à l'extrémité distale de la prothèse dépassent axialement par rapport au cathéter-poussoir, ce bouchon étant conçu de manière à assurer l'étanchéité entre les extrémités distales du cathéter-poussoir et de la capsule.

21. Instrumentation suivant l'une quelconque des revendications 18 à 20, caractérisée en ce que la capsule (32) est conçue pour être fendue longitudinalement, de manière à permettre le dégagement de l'extrémité distale de la prothèse (1 de la capsule (32) après la mise en place de cette prothèse (1) dans l'aorte (14).

22. Instrumentation suivant l'une quelconque des revendications 8 à 21, caractérisée en ce qu'elle comprend un ballon aortique pouvant être gonflé à l'intérieur de la prothèse (1) après la mise en place de cette dernière.

23. Instrumentation suivant l'une quelconque des revendications 8 à 22, caractérisée en ce que la gaine précitée (23) contient une prothèse intra-aortique (1) repliée radialement sur deux cathéters-poussoirs (24, 25) s'étendant à l'intérieur de la prothèse (1), cette dernière étant enserrée par la capsule (32) sur l'extrémité distale des cathéters-poussoirs (24-25), les guides (21, 22) étant introduits dans les cathéters-poussoirs (24, 25) et le piston (26) s'appuyant sur l'extrémité proximale de la prothèse (1), les guides (21, 22) dépassent aux deux extrémités de la gagne (23), les cathéter-poussoirs (24, 25) et la tige du piston (26) dépassent à l'extrémité proximale de la gaine (23).

## Claims

1. Intra-aortic prosthesis (1) made of a flexible, medically acceptable material, for treatment of an aneurysm (17) of the abdominal aorta (14) by the endovascular route, in particular at the site of the aortic intersection, comprising a tube (2) bifurcating at one of its extremities into two tubular branches (3) and (4) with cross-section smaller than that of said tube (2) and a cylindrically-shaped lattice (11) made of a memory metal and having a structure enabling it to be self-expanding within the tube (2), characterised in that two continuous stiffening bands (6) and (7) extend parallel to the axis of the tube (2) on the outside lateral sides, diametrically opposite to said axis, essentially over the whole length, and also extend essentially over the whole length of said two branches (3) and (4), said stiffening bands (6) and (7) having eyes (8) and (9) at each of their ends, into which suspension wires (41) and traction wires (40) respectively can be inserted.

2. Prosthesis (1) according to claim 1, characterised in that the above-mentioned stiffening bands (6) and (7) are formed of metal strips.

3. Prosthesis (1) according to either of claims 1 or 2, characterised in that an expandable crampon (10) is fixed at the proximal end of each of the stiffening bands (6) and (7), on the outside of said stiffening bands, oriented towards the opposite or distal end of said stiffening bands (6) and (7).

4. Prosthesis according to any of claims 1 to 3, characterised in that the stiffening bands (6) and (7) have openings along essentially their whole length.

5. Prosthesis according to any of claims 1 to 4, characterised in that the stiffening strips are sewn onto the tube (2) and the two tubular branches (3) and (4).

6. Prosthesis according to any of claims 1 to 5, characterised in that the above-mentioned lattice (11) is fixed to the tube (2) only at the point on the internal wall of said tube opposite the stiffening bands (6) and (7).

7. Prosthesis according to any of claims 1 to 6, characterised in that at least the branches (3) and (4) are essentially non-extensible, while their free end has a radially extensible annular zone (5).

8. Surgical instrumentation comprising means permitting the placing of an intra-aortic prosthesis according to any of claims 1 to 7, characterised in that it comprises at least one of the following instruments:
(a) an insertion sleeve (19), preferably pre-formed in a double curve, essentially "S"-shaped, destined to be engaged from an access made in the left subclavian artery (20) leading towards the aorta (14) and matching the configuration of the left subclavian-aortic confluent; (b) at least one guide (21) and (22) made of a radio-opaque material and preferably covered with a substance having lubricating properties in the presence of moisture; (c) a semi-rigid sheath (23) destined to engage in the above-mentioned insertion sleeve and the aorta (14) up to proximity with the ostium of the renal arteries; (d) at least one pusher catheter (24, 25); and (e) a piston (26) whose external section corresponds essentially to the interior section of said sheath (23), where said piston is able to rest on the prosthesis (1) engaged in said sheath (23) and is fitted with a semi-rigid control rod (27) which can operate the piston (26).

9. Instrumentation according to claim 8, characterised in that the insertion sleeve (19) is made of polytetrafluoroethylene.

10. Instrumentation according to either of claims 8 and 9, characterised in that it comprises a mandrill (28) made of a flexible material and destined to be mounted in the above-mentioned insertion sleeve (19), where the distal end of said mandrill (28) is rounded and is pierced so as to be able to slide on a radio-opaque guide (38) while assuring the necessary sealing.

11. Instrument according to any of claims 8 to 10, characterised in that it comprises a valve (29) destined to engage in the proximal end of the sleeve (19) and having passages for the above-mentioned rod (27) of the piston (26) and the pusher catheter (24, 25).

12. Instrumentation according to any of claims 8 to 11, characterised in that for each of the iliac arteries (15, 16) it comprises an insertion tube (30) with a French 9 valve for extracting the above-mentioned guides inserted into the aorta (14) via the subclavian artery (20).

13. Instrumentation according to any of claims 8 to 12, characterised in that the sheath (23) is made of semi-rigid polytetrafluoroethylene.

14. Instrumentation according to any of claims 8 to 13, characterised in that the distal end of the sheath (23) is provided with a radiological mark (39).

15. Instrumentation according to any of claims 8 to 14, characterised in that a conduit (31) connected to a catheter is incorporated in the wall of the sheath (23), permitting the creation of radiological opacity above the piston (26) and the prosthesis (1) which it contains.

16. Instrumentation according to any of claims 8 to 15, characterised in that the pusher catheter (24-25) is arranged so as to be able to serve as a conduit for the above-mentioned guide (21, 22).

17. Instrumentation according to any of claims 8 to 16, characterised in that it comprises two pusher catheters (24, 25) destined to be inserted into each of the branches (3) and (4) of the above-mentioned prosthesis (1).

18. Instrumentation according to any of claims 8 to 17, characterised in that the distal end of the pusher catheter (24, 25) is provided with a capsule (32) destined to accept the distal end of the prosthesis (1).

19. Instrumentation according to claim 18, characterised in that the capsule (32) is formed by a tubular fitting extending over a certain distance laterally and externally around the distal end of the pusher catheter (24, 25) so as to enable the distal end of the prosthesis to be held in the space (33) created between the pusher catheter (24, 25) and the capsule (32).

20. Instrumentation according to claim 19, characterised in that it comprises an annular plug (34) with rounded distal end, destined to keep the capsule (32) essentially coaxial around the distal end of the pusher catheter (24, 25), and to keep the traction wires (40) fixed at the distal end of the prosthesis projecting axially with respect to the pusher catheter, said plug being designed to assure sealing between the distal ends of the pusher catheter and the capsule.

21. Instrumentation according to any of claims 18 to 20, characterised in that the capsule (32) is designed to be split longitudinally, so as to free the distal end of the prosthesis (1) from the capsule (32) after said prosthesis (1) has been positioned in the aorta (14).

22. Instrumentation according to any of claims 8 to 21, characterised in that it comprises an aortic balloon which can be expanded inside the prosthesis (1) after said prosthesis has been positioned.

23. Instrumentation according to any of claims 8 to 22, characterised in that the above-mentioned sheath (23) contains an intra-aortic prosthesis (1) folded radially on two pusher catheters (24, 25) extending inside said prosthesis (1), said prosthesis being clamped by the capsule (32) on the distal end of the pusher catheters (24, 25), where the guides (21, 22) are inserted into said pusher catheters (24, 25) and the piston (26) rests on the proximal end of the prosthesis (1), the guides (21, 22) projecting beyond the two ends of the sheath (23) and the pusher catheters (24, 25) and the piston rod (26) projecting beyond the proximal end of the sheath (23).

## Patentansprüche

1. Intraaortale Prothese (1), gefertigt aus flexiblem Material und medizinisch unbedenklich, zur Behandlung eines Aneurysma (17) der Aorta abdominalis (14) auf endovaskulärem Wege, vor allem im Bereich des Aortakreuzpunktes, bestehend aus einer Tube (2), die an einem ihrer Enden in zwei Röhren (3) und (4) mit kleinerem Querschnitt als dem der Tube (2) verzweigt, und einem Flechtwerk (11) in zylindrischer Form, daß aus einem Metall mit Formerinnerungsvermögen gefertigt ist und eine Struktur aufweist, durch die es in der Lage ist, sich in der Tube (2) selbst auszubreiten, dadurch gekennzeichnet, daß zwei durchgehende Versteifungsstreifen (6) und (7) parallel zur Mittellinie der Tube (2) verlaufen, an den äußeren Seiten und diametral entgegengesetzt zu letzterer, über deutlich deren gesamte Länge, die sich gleichzeitig über deutlich die gesamte Länge der röhrenförmigen Verzweigungen (3) und (4) erstrecken, wobei die genannten Versteifungsstreifen (6) und (7) an jedem ihrer Enden Ösen (8) und (9) aufweisen, in denen Aufhänge- (41) beziehungsweise Zugdrähte (40) befestigt werden können.

2. Prothese (1) nach Anspruch 1, dadurch gekennzeichnet daß die vorbezeichneten Versteifungsstreifen (6) und (7) aus Metallplättchen bestehen.

3. Prothese (1) nach dem einen oder dem anderen der Ansprüche 1 und 2, dadurch gekennzeichnet, daß am nächstgelegenen Ende eines jeden der Versteifungsstreifen (6) und (7), im äußeren Bereich der beiden letztgenannten und in Nähe der Ösen (8) eine Klammer (10) befestigt ist, die seitlich dehnbar und auf das entgegengesetzte oder entferntere Ende der genannten Versteifungsstreifen (6) und (7) ausgerichtet ist.

4. Prothese (1) nach einem beliebigen der Ansprüche 1 bis 3, dadurch gekennzeichnet, daß die Versteifungsstreifen (6) und (7) über deutlich ihre gesamte Länge hinweg durchbrochen sind.

5. Prothese (1) nach einem beliebigen der Ansprüche 1 bis 4, dadurch gekennzeichnet, daß die Versteifungsstreifen (6) und (7) an der Tube (2) und den beiden röhrenförmigen Verzweigungen (3) und (4) angeheftet sind.

6. Prothese (1) nach einem beliebigen der Ansprüche 1 bis 5, dadurch gekennzeichnet, daß das vorgenannte Flechtwerk (11) an der Tube (2) nur an der inneren Seitenwand letzterer gegenüber den Versteifungsstreifen (6) und (7) befestigt ist.

7. Prothese (1) nach einem beliebigen der Ansprüche 1 bis 6, dadurch gekennzeichnet, daß zumindest die Streifen (3) und (4) deutlich unausdehnbar sind, wogegen ihr freies Ende einen ringförmigen Bereich (5) aufweist, der radial ausdehnbar ist.

8. Chirurgische Vorrichtung, die Mittel zur Positionierung einer intraaortalen Prothese nach einem der Ansprüche 1 bis 7 enthält, dadurch gekennzeichnet, daß sie mindestens einen der folgenden Vorrichtungsteile enthält:
(a) eine Einführhülse (19), vorzugsweise durch zwei Bögen in deutlicher S-Form vorgeformt, die dazu bestimmt ist, über einen praktischen Zugang in der linken Arteria subclavia (20) in Richtung Aorta (14) eingesetzt zu werden, indem die Konfiguration des Confluens im Bereich der linken Aorta subclavia angepaßt wird, (b) mindestens eine Führung (21) und (22), die aus einem strahlenundurchlässigen Material gefertigt und vorzugsweise mit einer Substanz bedeckt ist, die bei Vorhandensein von Feuchtigkeit, die Eigenschaften eines Schmiermittels annimmt, (c) eine halbfeste Umhüllung (23), die dazu bestimmt ist, bis in Nähe des Ostiums der Arteriae renalis in die oben genannte Einführungshülse und die Aorta (14) eingesetzt zu werden, (d) mindestens eine Einschubsonde (24, 25) und (e) einen Kolben (26), dessen äußerer Querschnitt deutlich dem Innenquerschnitt der Umhüllung (23) entspricht, wobei der Kolben auf der Prothese (1) ruhen kann, die in die Umhüllung (23) eingeführt ist und die mit einer halbfesten Steuerkolbenstange (27) zur Bewegung des Kolbens (26) versehen ist.

9. Vorrichtung nach Anspruch 8, dadurch gekennzeichnet, daß die Einführungshülse (19) aus Polytetrafluorethylen besteht.

10. Vorrichtung nach dem einem oder dem anderen der Ansprüche 8 und 9, dadurch gekennzeichnet, daß sie einen Dorn enthält, der aus einem flexiblen Material besteht und dazu bestimmt ist, in die vorbezeichnete Einführhülse (19) eingebaut zu werden, wobei das entferntere Ende dieses Dorns abgerundet ist und durchbohrt ist, um auf einer strahlenundurchlässigen Führung (38) gleiten zu können und dabei voll und ganz die notwendige Dichtheit zu gewahrleisten.

11. Vorrichtung nach einem beliebigen der Ansprüche 8 bis 10, dadurch gekennzeichnet, daß sie über ein Ventil (29) verfügt, das dazu bestimmt ist, in das näher gelegene Ende der Einführhülse (19) eingesetzt zu werden, und das über Durchgänge für die vorbezeichnete Stange (27) des Kolbens (26) und die Einschubsonde (24, 25) verfügt.

12. Vorrichtung nach einem beliebigen der Ansprüche 8 bis 11, dadurch gekennzeichnet, daß sie für jede der Iliakalarterien (15, 16) ein Einführungsrohr (30) mit French 9-Ventil zur Rückholung der vorgenannten Führungen über die linke Arteria subclavia (20) in der Aorta (14) enthält.

13. Vorrichtung nach einem beliebigen der Ansprüche 8 bis 12, dadurch gekennzeichnet, daß die Umhüllung aus halbfestem Polytetrafluorethylen gefertigt ist.

14. Vorrichtung nach einem beliebigen der Ansprüche 8 bis 13, dadurch gekennzeichnet, daß das entferntere Ende der Umhüllung (23) über eine Aufnahmevorrichtung für radiologische Gerätschaften verfügt.

15. Vorrichtung nach einem beliebigen der Ansprüche 8 bis 14, dadurch gekennzeichnet, daß eine mit einer Sonde verbundene Leitung (31) in die Wandung der Umhüllung (23) eingebracht ist, wodurch der Raum oberhalb des Kolbens (26) und der Prothese (1), die sie enthält, für Röntgenstrahlen undurchlässig gemacht werden kann.

16. Vorrichtung nach einem beliebigen der Ansprüche 8 bis 15, dadurch gekennzeichnet, daß die Einschubsonde (24-25) auf eine Weise beschaffen ist, daß sie als Leiteinrichtung für die vorgenannte Führung (21, 22) dienen kann.

17. Vorrichtung nach einem beliebigen der Ansprüche 8 bis 16, dadurch gekennzeichnet, daß sie zwei Einschubsonden (24, 25) enthält, die beide jeweils dazu bestimmt sind, in eine der Abzweige (3) und (4) der vorgenannten Prothese (1) eingeführt zu werden.

18. Vorrichtung nach einem beliebigen der Ansprüche 8 bis 17, dadurch gekennzeichnet, daß das entferntere Ende der Einschubsonde (24, 25) mit einer Kapsel (32) versehen ist, die dazu bestimmt ist, das entferntere Ende der Prothese (1) aufzunehmen.

19. Vorrichtung nach Anspruch 18, dadurch gekennzeichnet, daß die Kapsel (32) durch eine Röhrendichtung gebildet wird, die sich in einer gewissen Entfernung um das entferntere Ende der Einschubsonde (24, 25) herum seitlich und äußerlich ausdehnt und somit ermöglicht, daß das entferntere Ende der Prothese in dem Raum (33) gehalten wird, der zwischen der Einschubsonde (24, 25) und der Kapsel (32) vorgesehen ist.

20. Vorrichtung nach Anspruch 19, dadurch gekennzeichnet, daß sie einen Ringstopfen am abgerundeten entfernteren Ende enthält, der dazu bestimmt ist, einerseits die Kapsel (32) deutlich koaxial um das entferntere Ende der Einschubsonde (24, 25) herum und andererseits Zugdrähte (40), die am entfernteren Ende der Prothese befestigt sind und im Verhältnis zur Einschubsonde axial verlaufen, zu halten, wobei der genannte Stopfen solcherart konstruiert ist, daß er die Dichtigkeit zwischen den entfernteren Enden des Einschubsonde und der Kapsel gewährleistet.

21. Vorrichtung nach einem beliebigen der Ansprüche 18 bis 20, dadurch gekennzeichnet, daß die Kapsel solcherart konstruiert ist, daß sie in Längsrichtung aufgespalten werden kann, damit auf diese Weise das entferntere Ende der Prothese (1) und der Kapsel (32) nach der Positionierung der genannten Prothese (1) in der Aorta (14) entfernt werden kann.

22. Vorrichtung nach einem beliebigen der Ansprüche 8 bis 21, dadurch gekennzeichnet, daß sie einen Aortaballon umfaßt, der im Inneren der Prothese (1) nach deren Positionierung prallgefüllt werden kann.

23. Vorrichtung nach einem beliebigen der Ansprüche 8 bis 21, dadurch gekennzeichnet, daß die vorbezeichnete Umhüllung (23) eine intraaortale Prothese (1) enthält, die radialförmig auf zwei Einschubsonden (24, 25) zusammengefaltet wird, die sich im Innern der Prothese (1) ausdehnen, wobei letztere durch die Kapsel (32) am entfernteren Ende der Einschubsonden (24-25) umschlossen wird, die Führungen (21, 22) in die Einschubsonden (24, 25) eingeführt werden und der Kolben (26) auf dem nächstgelegenen Ende der Prothese (1) zu ruhen kommt, die Führungen (21, 22) an den beiden Enden der Umhüllung (23) vorbeilaufen, die Einschubsonden (24, 25) und die Kolbenstange (26) am nächstgelegenen Ende der Umhüllung (23) vorbeilaufen.
